Europäisches Patentamt

European Patent Office

Office européen des brevets

(11) Veröffentlichungsnummer: **0 358 763**
**A1**

(12) **EUROPÄISCHE PATENTANMELDUNG**
veröffentlicht nach Art. 158 Abs. 3
EPÜ

(21) Anmeldenummer: 88906826.8

(51) Int. Cl.5: **C07C 11/08 , C07C 2/30**

(22) Anmeldetag: 26.05.88

(86) Internationale Anmeldenummer:
PCT/SU88/00129

(87) Internationale Veröffentlichungsnummer:
WO 89/00553 (26.01.89 89/03)

(30) Priorität: 13.07.87 SU 4267266

(43) Veröffentlichungstag der Anmeldung:
21.03.90 Patentblatt 90/12

(84) Benannte Vertragsstaaten:
AT BE CH DE FR GB IT LI LU NL SE

(71) Anmelder: INSTITUT KHIMICHESKOI FIZIKI
AKADEMII NAUK SSSR
ul. Kosygina, 4
Moscow, 117977(SU)

Anmelder: GROZNENSKY FILIAL
OKHTINSKOGO NAUCHNO-
PORIZVODSTVENNOGO OBIEDINENIA
"PLASTPOLIMER"

Grozny, 364048(SU)

(72) Erfinder: SERGIENKO, Galina Stepanovna
ul. Rabochaya, 64-43
Grozny, 364051(SU)
Erfinder: ZHUKOV, Viktor Ivanovich
ul. Neftezavodskaya, 4-1
Grozny, 364007(SU)
Erfinder: BELOV, Gennady Petrovich
Shkolny bulvar, 5-53 Moskovskaja obl.
Chernogolovka, 142432(SU)

Erfinder: DYACHKOVSKY, Fridrikh
Stephanovich
ul. Kosygina, 6-9
Moscow, 117334(SU)
Erfinder: IVANCHEV, Sergei Stepanovich
ul. Nalichnaya, 36-37
Leningrad, 199226(SU)
Erfinder: GERMASHEV, Anatoly Ivanovich
kvartal 175a, 5-5 Stavropolsky krai
Budennovsk, 357920(SU)
Erfinder: PETROV, Jury Maximovich
mikroraion 7, 17-37 Stavropolsky krai
Budennovsk, 357920(SU)
Erfinder: LAZUTIN, Valery Ignatievich
ul. Timiryazeva, 77
Grozny, 364048(SU)
Erfinder: YATSENKO, Valery Alexeevich
pr. Lenina, 117-85
Grozny, 364048(SU)
Erfinder: GABUTDINOV, Malik Salikhovich
ul. Okolnaya, 94a-2-41
Kazan, 420015(SU)

(74) Vertreter: von Füner, Alexander, Dr. et al
Patentanwälte v. Füner, Ebbinghaus, Finck
Mariahilfplatz 2 & 3
D-8000 München 90(DE)

(54) **VERFAHREN ZUR HERSTELLUNG VON BUTEN-1.**

(57) Die Herstellung von Buten-(1) erfolgt durch Dimerisation von Äthylen in Gegenwart eines katalytischen Systems Titantetraalkoxid-Trialkylaluminium in einem kohlenwasserstoffhaltigen Lösungsmittel und durch die anschließende Rektifikation der Reaktionsmasse, die bei der Dimerisation in Gegenwart einer Verbindung entsteht, die aus einer Gruppe gewählt wird, die aliphatische einund zweiwertige Alkohole,

aliphatische und zyklische Äther, aliphatische Ketone
und Karbonsäureamide enthält

# VERFAHREN ZUR HERSTELLUNG VON BUTEN-(1)

## Gebiet der Technik

Die Erfindung bezieht sich auf chemische und petrolchemische Industrie und insbesondere auf ein Verfahren zur Herstellung von Buten-(1).

## Vorhergehender Stand der Technik

Bekannt ist ein Varfahren zur Dimerisation von Äthylen unter Einwirkung eines katalytischen Systems - Diisobutylaluminiumchlorid - Nickeloleat bei unterschiedlichen Bedingungen unter Anfallen von Buten-(1). Der maximale Gehalt an Buten-(1) in dem herzustellenden Dimerisat beträgt 59,1 Masse% und seine Zusammensetzung ist dabei wie folgt: trans-Buten-(1) - 23,9 Masse%, cis-Buten-(2) - 12,0 Masse%, Buten-(1) - 59,1 Masse%, n-Buten - 5 Masse%. Bei der Realisierung dieses Verfahrens ist die Buten-(1)-bezogene Selektivität niedrig, es entsteht eine große Menge von Nebenprodukten, insbesondere von Butenen-(2) im Dimerisat, während für Polymerisation und Kopolymerisation Buten-(1) erforderlich ist, das nicht über 0,2 Masse% Butene-(2) enthält (V.Sh.Feldbljum "Dimerisation und Disproportionierung von Olefinen", 1978, Chemie, (Moskau), S. 38).

Bekannt ist ebenfalls ein Verfahren zur Dimerisation von Olefinen in Gegenwart eines Katalysators, der eine Schicht aus Nickelkomplexen auf einem festen Träger darstellt, der sich aus $SiO_2$, Aluminiumsilikat beziehungsweise aus ihrem Gemisch zusammensetzt. Das herzustellende Dimerisat setzt sich aus 70 Masse% Buten-(1) und 30 Masse% Butenen-(2) zusammen (US, A, 4000211).

Bei der Durchführung dieses Verfahrens wird ein kostspielieger Katalysator verwendet, dessen Buten-(1)-bezogene Selektivität niedrig ist, das anfallende Dimerisat enthält eine große Anzahl von Butenen-(2).

Bekannt ist die Herstellung von Buten-(1) durch Dimerisation von Äthylen unter Einwirkung eines Katalysators, Titanalkoxids $Ti(OR)_4$ - Aluminiumtrialkyl $AlR_3$ im Äthermedium (Kinetik und Katalyse, Band 19, Ausgabe 1, 1978, P.E. Matkovski und andere "Dimerisation von Äthylen", S.263).

Die Verwendung von Äthern als Reaktionsmedium ist jedoch in der Industrie infolge der Kostspieligkeit und ihrer starken narkotischen Wirkung nicht anwendbar.

Bei der Herstellung von Buten-(1) mittels Dimerisation von Äthylen an einem katalytischen System $Ti(OR)_4$/ /$AlR_3$ entsteht außerdem das Problem sowohl der Ausscheidung von Buten-(1) aus dem Produktengemisch, die bei der Dimerisation von Äthylen anfallen (Buten-(1), Hexene, Oktene, ein Polymer, ein Katalysator) als auch das Problem der Trennung und Isolierung des Katalysators, der sich im Reaktionsgemisch vollständig auflösen kann. In diesem Falle ist die Anwendung von zwei Methoden möglich:

1) Entfernung des Katalysators vor Destillation von Kohlenwasserstoffen entweder durch Senkung des Auflösevermögens und durch Filtration oder Zentrifugierung, oder durch Zweiphasen-Extraktion, beispielsweise mit Wasser;

2) dierekte Destillation von Kohlenwasserstoffen (nichtumgesetztes Äthylen, Buten-(1), Hexene und andere Oligomere), bei der der Katalysator am Boden des Apparates für Destillation bleibt; er bleibt löslich, ist aber in Oligomeren konzentriert (CS, B, 187822).

Vom ökonomischen Standpunkt aus ist dieses Verfahren vorzuziehen, da es erlaubt, die Zwischenstufe der Zweiphasen-Waschung beziehungsweise der Trennung von festen Teilchen wegzulassen; der Rückstand fällt in geringen Mengen an und läßet sich, beispielsweise durch Glühen leicht entfernen.

Es wurde gleichzeitig in der Praxis nachgewiesen, daß die Temperatur bei dem Eindampfen relativ erhöht bleibt, die Erhöhung der Temperatur ist für die Erreichung der besten Konzentration des Katalysators erforderlich, sie ruft jedoch sekundäre Reaktionen der Isomerisation und Polymerisation von Buten-(1) hervor.

Bekannt ist ein Verfahren, bei dem man Buten-(1)
durch Dimerisation von Äthylen in Gegenwart eines katalytischen Systems $Ti(OC_4H_9)_4/AlR_3$ bei kontinuierlicher Zuführung von Äthylen, eines kohlenwasserstoffhaltigen Lösungsmittels und Lösungen der Komponenten des
Katalysators in einem kohlenwasserstoffhaltigen Lösungsmittel (Fr, B, 2581381) herstellt.

Das aus dem Reaktor auszuführende Reaktionsmasse
leitet man der Fraktionierung (Destillation oder Rektifikation) zu.

Bei der Durchführung dieses Verfahrens erfolgt die
Verringerung der Qualität von Buten-(1) infolge der
Entstehung von Buten-(2). Das letztere tritt bei der
Fraktionierung infolge des Isomerisation von Buten-(1)
an Produkten eines katalytischen Systems auf. Je höher
dabei die Temperatur in der Rektifikations- oder Destillationskolonne und je größer die durchschnittliche Verweilzeit der Reaktionsmasse in der Kolonne ist, desto intensiver sich die Isomerisation vollzieht, desto höher der
Gehalt des Butens-(1) an Buten-(2) ist. In dem Temperaturenbereich von 90 bis 120 $^{\circ}C$ kann der Gehalt des Butens-(1) an Buten-(2) von 2 bis 4 Vol% betragen, was
den Anforderungen nicht entspricht, die an den Rohstoff
gestellt werden, der für Homo- und Kopolymerisation eingesetzt wird.

Zur teilweisen Unterdrückung der Reaktion der Isomerisation von Buten-(1) zum Buten-(2) wird vor Beginn
der Rektifikation ein Modifizierungsmittel aus der Klasse der Amine, beispielsweise, Zyklohexylamin, 2-Äthylhexylamin, Dibutylamin, dem Reaktionsgemisch zugesetzt.
Die Menge des zuzusetzenden Amins variiert man im Bereich des molaren Verhältnisses Amin: Titan (das im Reaktionsgemisch enthalten ist) in einem Bereich von 0,1:1 bis 10:1, vorzugsweise von 0,3:1 bis 2:1.

Die Verwendung als Modifizierungsmittel der Amine von Verbindungen, die in der Regel sehr toxische Verbindungen (die zulässigen Grenzkonzentrationen für die überwiegende Anzahl der Amine, die in FR, B, 2581381 genannt sind, liegen bedeutend unter 10 mg/m$^3$) darstellen, hemmt die Anwendung des genannten Verfahrens unter den großtechnischen Bedingungen.

Offenbarung der Erfindung

Der vorliegenden Erfindung liegt die Aufgabe zugrunde, durch Änderung der Klasse der als Modifizierungsmittel einzusetzenden Verbindungen, die die Reaktion der Isomerisation von Buten-(1) zum Buten-(2) unterdrükken, ein solches Verfahren zur Herstellung von Buten-(1) zu entwickeln, welches es ermöglicht, ein Endprodukt mit einem höheren Reinheitsgrad unter Einsatz von weniger toxischen Verbindungen herzustellen.

Diese Aufgabe wird dadurch gelöst, daß man im Verfahren zur Herstellung von Buten-(1), das in der Dimerisation von Äthylen, die in Gegenwart eines katalytischen Systems Titantetralkoxid - Trialkylaluminium in einem kohlenwasserstoffhaltigen Lösungsmittel erfolgt, und in der anschließender Rektifikation der Reaktionsmasse in Gegenwart eines Modifizierungsmittels besteht, erfindungsgemäß als Modifizierungsmittel eine Verbindung verwendet, die aus der Gruppe aliphatischer ein- oder zweiwertiger Alkohole, aliphatischer Äther oder zyklischer Äther, alyphatischer Ketone, Karbonsäureamide gewählt wird.

Auf Grund der angemeldeten Erfindung wurde es möglich, den Reinheitsgrad des herzustellenden Buten-(1) im Vergleich zum Endprodukt, das in Übereinstimmung mit dem in FR, B, 2581381 angemeldeten Verfahren hergestellt wird, auf das 2,0 bis 9,3fache zu erhöhen, ohne dabei toxische Verbindungen einzusetzen.

Gemäß der angemeldeten Erfindung ist es zweckmäßig, bei der Verwendung aliphatischer ein- oder zweiwertiger Alkohole, aliphatischer Äther oder zyklischer Äther, aliphatischer Ketone als Modifizierungsmittel die Rektifikation bei einem Molverhältnis des Modifizierungsmittels zum Trialkylaluminium des katalytischen Systems gleich 1 bis 3 durchzuführen.

Auf Grund der angemeldeten Erfindung ist es möglich, die Herstellung von Buten-(1) unter Verwendung von Verbindungen vorzunehmen, deren zulässige Grenzkonzentration von 200 bis 500 mg/m$^3$ beträgt.

Damit es möglich wird, das angemeldete Verfahren in Gegenwart von weniger flüchtigen Verbindungen in Übereinstimmung mit der beanspruchten Erfindung durchzuführen, soll zweckmäßigerweise bei der Verwendung der Karbonsäureamide als Modifizierungsmittel die Rektifikation bei einem Molverhältnis des Modifizierungsmittels zum Trialkylaluminium des katalytischen Systems gleich 0,25 bis 3,00 vorgenommen werden.

Beste Ausführungsvariante der Erfindung

Weitere Ziele und Vorteile der vorliegenden Erfindung werden aus der nachstehenden ausführlichen Beschreibung des Verfahrens zur Herstellung von Buten-(1) und der Ausführungsbeispiele dieses Verfahrens ersichtlich.

Das in der vorliegenden Erfindung angemeldete Verfahren zur Herstellung von Buten-(1) besteht aus folgenden Hauptstufen:

- katalytische Dimerisation von Äthylen;
- Desaktivierung des Katalysators;
- Ausscheidung des Buten-(1) aus den Produkten der Dimerisation von Äthylen.

Die Stufe der katalytischen Dimerisation von Äthylen wird in einem Reaktor durchgeführt, der mit einem mechanischen Mischer versehen ist oder ohne desselben und folgendes vorsicht: eine periodische oder kontinuirliche Zuführung einem Reaktor von Lösungen der Komponenten des Katalysators in einem kohlenwasserstoffhaltigen
Lösungsmittel oder ihres Gemisches, kontinuierliche
Zuführung von Äthylen, periodische Ableitung von Dimerisationsprodukten aus dem Reaktor.

Als Katalysator verwendet man ein katalytisches Zweikomponenten-System, das sich aus Verbindungen des Titantetraalkoxids allgemeiner Formel $Ti(OR)_4$ und des Trialkylaluminiums allgemeiner Formel $AlR'_3$ zusammensetzt, worin R und R' - Alkylreste sind, die von 1 bis 6 Kohlestoffatome enthält.

Das Molverhältnis der Komponenten des Katalysators $AlR'_3$ $Ti(OR)_4$ kann von 1 bis 100 betragen, vorzuziehen sind die Verhältnisse von 2,5 bis 4. Diese Verbindungen werden in der Regel in Form von verdünnten Lösungen verwendet. Als Lösungsmittel dafür können aliphatische (Butan, Pentan, Hexan, Heptan), aromatische (Benzol, Toluol) Kohlenwasserstoffe oder Olefine (Buten-(1), Pentene, Hexene) oder ihre Gemische eingesetzt werden.

Die Stufe der katalytischen Dimerisation von Äthylen erfolgt bei einer Temperatur von 20 bis 100°C, vorzugsweise von 50 bis 80°C.

Die Verweilezeit des Katalysators im Reaktor kann von einigen wenigen Minuten bis zu mehreren Stunden in Abhängigkeit von den Bedingungen der Führung der Dimerisationsstufe schwanken.

Die im Reaktor entstandenen Produkte der Dimerisation von Äthylen werden zusammen mit dem Lösungsmittel aus dem Reactor in einen Zwischenbehälter abgeleitet, in dem man die Desaktivierung des Katalysators durch Zugabe der in dieser Erfindung vorgeschlagenen Midifizierungsmittel (aliphatische ein- oder zweiwertige Alkohole, aliphatische Äther oder zyklische Äther, aliphatische Ketone oder Karbonsäureamide) dem Reaktionsgemisch vornimmt.

Eine derartige Desaktivierung bewirkt die Unterdrükkung der Isomerisation von Buten-(1) zum Buten-(2) in der anschließenden Ausscheidungsstufe von Buten-(1) aus dem Reaktionsgemisch. Die Ausscheidung von Buten-(1) (Rektifikation) kann in einer Rektifikationskolonne erfolgen, wobei die Temperatur in der Kolonnenküpe in einem Bereich von 80 bis 100°C gehalten wird.

Als einwertigen Alkohol kann man Äthyl-, Propyl-, Butyl-, Amylalkohole, vorzugsweise Isopropylalkohol einsetzen (zulässige Grenzkonzentration beträgt von 10 bis 150 mg/m$^3$).

Als zweiwertiger Alkohol HOROH kann, beispielsweise, Diäthylenglykol, Butandiol (zulässige Grenzkonzentration beträgt von 260 bis 500 mg/m$^3$) sowie Äther der zweiwertigen Alkohole, beispielsweise Diäthylenglykoldimethyläther (zulässige Grenzkonzentration beträgt 80 mg/m$^3$) verwendet werden.

Als Äther können, beispielsweise, Diäthyl-, Isopropyl-, und Diisoamyläther, vorzugsweise Diisoamyläther, verwendet werden.

Als zyklische Äther können, beispielsweise, Tetrahydrofuran, Dioxan (zulässige Grenzkonzentration beträgt von 10 bis 100 mg/m$^3$) verwendet werden.

Als Ketone können, beispielsweise Methyläthylketon und Azeton (zulässige Grenzkonzentration beträgt 200 mg/m$^3$) verwendet werden.

Als Karbonsäureamide kann, beispielsweise, Dimethylformamid (zulässige Grenzkonzentration beträgt 10 mg/m$^3$) verwendet werden.

Aus praktischen Erwägungen heraus ist es wünschenswert, Modifizierungsmittel mit einer relativ niedrigen Dampfspannung bei einer Rektifikationstemperatur (von 90 bis 150°C) zu wählen.

In Übereinstimmung mit der angemeldeten Erfindung wird das Modifizierungsmittel in einer solchen Menge eingesetzt, daß sein maximales Molverhältnis zum Trialkylaluminium - AlR$_3$, das in der Reaktionsmasse enthalten ist, nicht über 3 beträgt.

Das minimale Molverhältnis Modifizierungsmittel: AlR$_3$ (bei Verwendung von ein- und zweiwertigen Alkoholen, von Äthern oder zyklischen Äthern, Ketonen) ist gleich 1 oder (bei Verwendung von Karbonsäureamiden) beträgt es 0,25.

Bei einem Gehalt an Komponenten in der in die Stufe der Desaktivierung eintretenden Reaktionsmasse in einem Verhältnis unter der angemeldeten unteren Grenze verringert sich der Gehalt an Buten-(2) im Buten-(1) nicht. Die Unterhaltung des Verhältnisses der genannten Komponenten oberhalb der oberen angemeldeten Grenze ist unzweckmäßig, weil dabei keine weitere Verbesserung des Buten-(1) erreicht wird. Das ist darauf zurückzuführen, daß bei der Verwendung des Modifizierungsmittels in den das minimale angemeldete Verhältnis nichtabsichernden Mengen das freie Trialkylaluminium in der Reaktionsmasse bleibt, das bei erhöhter Temperatur mit der titanhaltigen Konponente des Katalysators zusammenwirkt und aktive Zentren bildet, die Buten-(1) zum Buten-(2) isomerisieren. Bei der Zuführung des Modifizierungsmittels in einer Menge, die ein oberhalb des angemeldeten Verhältnisses liegendes Verhältnis bewirkt, wird die Verbesserung des Reinheitsgrades des Buten-(1) nicht erreicht, weil die Wahrscheinlichkeit der Entstehung von Isomerisationszentren bereits bei dem oberen Wert von 3 auf Null gebracht wird, und eine weitere Erhöhung dieses Wertes lediglich zum Mehrverbrauch des Modifizierungsmittels und zur Erhöhung der Herstellungskosten von Buten-(1) führt.

Der Einsatz der beanspruchten Modifizierungsmittel in den genannten Verhältnissen zum Trialkylaluminium erhöht auf das 2 bis 9,3 fache den Reinheitsgrad des auszuscheidenden Buten-(1) (auf Kosten der Reaktionsverringerung der Isomerisation des Buten-(1) zum Buten-(2)).

Die Verwendung der beanspruchten Verbindungen als Modifizierungsmittel führt außerdem zusätzlich zu folgenden positiven Effekten: infolge der Verringerung des Verbrauchs an Buten-(1) für die Isomerisation desselben zum Buten-(2) steigert die Ausbeute an Buten-(1) bei seiner Fraktionierung; es wird die Toxizität der Herstellung verringert, weil die zum Einsatz kommenden Modifizierungsmittel weniger toxisch sind (auf das 3 bis 10 fache) im Vergleich zu den Modifizierungsmitteln (Aminen) sind, die in dem in FR, B, 2581381 angemeldeten Verfahren verwendet werden.

Zur besseren Erläuterung der vorliegenden Erfindung werden nachstehende Beispiele für seine konkrete Ausführung angeführt.

Beispiel 1

Lösungen $Ti(OC_4H_9)_4$ und $Al(iso-C_4H_9)_3$ in Pentan, Pentan als Lösungsmittel und Äthylen werden kontinuierlich einem Reaktor mit einem Volumen von 0,5 m$^3$ zugeführt. Die Konzentration des Katalysators beträgt 1 g/l. Die Dimerisation führt man bei einer Temperatur von $50^\circ$C durch. Die Reaktionsmasse, die aus dem Reaktor abgeleitet wird, führt man der Destillation zu, indem man dieser Masse Isopropylalkohol in einem Verhältnis 1 Mol je 1 Mol $Al/C_4H_9/_3$ vorher zusetzt, der in der Reaktionsmasse enthalten ist. Die Zusammensetzung des Destillats nach der Destillation ist wie folgt, %: Äthylen - 9,96; Butan - 0,42; Buten -(1) - 81,56; Buten-(2) - 0,44; Hexene - 5,53; Lösungsmittel - 2,09.

Beispiele 2 bis 10

Die Herstellung von Buten-(1) erfolgt ähnlicherweise wie in Beispiel 1. Die Rektifikation der Reaktionsmasse führt man jedoch in Gegenwart von Methyläthylketon (Beispiele 2,3,4), von Isoamylalkohol (Beispiele 5 und 6), von Diäthylenglykol (Beispiel 7), von Äthylenglykol (Beispiel 8), von Azetamid (Beispiel 9), von Benzamid (Beispiel 10).

Die Zusammensetzung des Destillats nach der Rektifikation ist in der Tabelle 1 angeführt.

Beispiel 11

Die Herstellung von Buten-(1) erfolgt ähnlicherweise wie in Beispiel 1 mit Ausnahme dessen, daß man $Al/iso-C_4H_9/_3$ verwendet und man in die aus dem Reaktor abzuleitende Reaktionsmasse den Isopropylalkohol in einem Verhältnis von 3 Mol Alkohol je 1 Mol $Al/iso-C_4H_9/_3$ einführt. Die Zusammensetzung des Destillats nach der Destillation ist wie folgt, %: Äthylen - 10,5; Butan - 0,40; Buten-(1) - 81,76; Buten-(2) - 0,24; Hexene - 5,10; Lösungsmittel - 2,00.

Beispiel 12

Die Herstellung von Buten-(1) erfolgt ähnlicherweise wie in Beispiel 1 mit Ausnahme dessen, daß man in die aus dem Reaktor abzuleitende Reaktionsmasse Diäthylenglykol in einem Verhältnis 1 Mol je 1 Mol $Al/C_2H_5/_3$ ein führt. Die Zusammensetzung des Destillats nach der Destillation ist wie folgt, %: Äthylen - 10,1; Butan - 0,45; Buten-(1) - 81,50; Buten-(2) - 0,50; Hexene - 5,75; Lösungsmittel - 1,98.

Beispiel 13

Die Herstellung von Buten-(1) erfolgte ähnlicherweise wie in Beispiel 11, man führt jedoch in die aus dem Reaktor abzuleitende Reaktionsmasse Diäthylenglykol in einem Verhältnis 3 Mol je 1 Mol $Al/iso-C_4H_9/_3$ ein. Die Zusammensetzung des Destillats nach der Destillation ist wie folgt, %: Äthylen - 10; Butan - 0,45; Buten-(1) - 81,80; Buten-(2) - 0,20; Hexene - 5,55; Lösungsmittel - 2,0.

Beispiel 14

Die Herstellung von Buten-(1) erfolgt ähnlicherweise wie in Beispiel 1 mit Ausnahme dessen, daß man in die aus dem Reaktor abzuleitende Reaktionsmasse Diisoamyläther in einem Verhältnis von 1 Mol je 1 Mol $Al/C_2H_5/_3$ einführt. Die Zusammensetzung des Destillats nach der Destillation ist wie folgt, %: Äthylen - 9,98; Butan - 0,41; Buten-(1) - 81,61; Buten -(2) - 0,49; Hexene - 5,43; Lösungsmittel - 2,08.

Beispiel 15

Die Herstellung von Buten-(1) erfolgt ähnlicherweise wie in Beispiel 1, man führt jedoch in die aus dem Reaktor abzuleitende Reaktionsmasse Diisoamyläther in einem Verhältnis von 3 Mol je 1 Mol $Al/C_2H_5/_3$ ein. Die Zusammensetzung des Destillats nach der Destillation ist wie folgt, %: Äthylen - 9,96; Butan - 0,42; Buten-(1) - 81,67; Buten-(2) - 0,15; Hexene - 5,51; Lösungsmittel - 2,29.

Beispiel 16

Die Herstellung von Buten-(1) erfolgt ähnlicherweise wie in Beispiel 2 mit Ausnahme dessen, daß man in die aus dem Reaktor abzuleitende Reaktionsmasse Dioxan in einem Verhältnis von 1 Mol je 1 Mol $Al/iso-C_4H_9/_3$ einführt. Die Zusammensetzung des Destillats nach der Destillation ist wie folgt, %: Äthylen - 9,90; Butan - 0,40; Buten-(1) - 81,49; Buten-(2) - 0,54; Hexene - 5,67; Lösungsmittel - 2,03.

Beispiel 17

Die Herstellung von Buten-(1) erfolgt ähnlicherweise wie in Beispiel 16, man führt jedoch Dioxan in einem Verhältnis von 3 Mol je 1 Mol $Al/iso-C_4H_9/_3$ ein. Die Zusammensetzung des Destillats nach der Destillation ist wie folgt, %: Äthylen - 9,96; Butan - 0,44; Buten-(1) - 81,70; Buten-(2) - 0,30; Hexene - 5,51; Lösungsmittel - 2,09.

Beispiel 18

Die Herstellung von Buten-(1) erfolgt ähnlicherweise wie in Beispiel 1, man führt jedoch in die aus dem Reaktor abzuleitende Reaktionsmasse Dimethylformamid in einem Verhältnis 0,25 Mol je 1 Mol $Al/C_2H_5/_3$ ein. Die Zusammensetzung des Destillats nach der Destillation ist wie folgt, %: Äthylen - 10,0; Butan - 0,40; Buten-(1) - 81,67; Buten-(2) - 0,35; Hexene - 5,58; Lösungsmittel - 2,00.

Beispiel 19

Die Herstellung von Buten-(1) erfolgt ähnlicherweise wie in Beispiel 1, man führt jedoch Dimethylformamid in einem Verhältnis 3 Mol je 1 Mol Al/iso-$C_4H_9/_3$ ein. Die Zusammensetzung des Destillats nach der Destillation ist wie folgt, %: Äthylen - 9,98; Butan - 0,44; Buten-(1) - 81,80; Buten-(2) - 0,1; Hexene - 5,56; Lösungsmittel - 2,12.

Beispiel 20

Die Herstellung von Buten-(1) erfolgt ähnlicherweise wie in Beispiel 1, man führt jedoch in die aus dem Reaktor abzuleitende Reaktionsmasse Methyläthylketon in einem Verhältnis von 1 Mol je 1 Mol Al/$C_2H_5/_3$ ein. Die Zusammensetzung des Destillats nach der Destillation ist wie folgt, %: Äthylen - 10,01; Butan - 0,42; Buten-(1) - 81,6; Buten-(2) - 0,4; Hexene - 5,57; Lösungs - mittel - 2,00.

Beispiel 21

Die Herstellung von Buten-(1) erfolgt ähnlicherweise wie in Beispiel 1, man führt lediglich Methyläthylketon in einem Verhältnis von 3 Mol je 1 Mol Al/iso-$C_4H_9/_3$ ein. Die Zusammensetzung des Destillats nach der Destillation ist wie folgt, %: Äthylen - 10,3; Butan - 0,43; Buten-(1) - 81,77; Buten-(2) - 0,25; Hexen - 5,18; Lösungsmittel - 2,07.

Beispiel 22

Die Herstellung von Buten-(1) erfolgt ähnlicherweise wie in Beispiel 1, man verwendet jedoch als Lösungsmittel Heptan und als Modifizierungsmittel - Azeton. Die Zusammensetzung des Destillats nach der Destillation ist wie folgt, %: Äthylen - 11,57; Butan - 0,40; Buten-(1) - 81,75; Buten-(2) - 0,25; Hexene - 5,08; Lösungsmittel - 0,95.

Beispiel 23

Die Herstellung von Buten-(1) erfolgt ähnlicherweise wie in Beispiel 1, man verwendet nur als Lösungsmittel eine Hexanfraktion und als Modifizierungsmittel - Azeton. Die Zusammensetzung des Destillats nach der Destillation ist wie folgt, %: Äthylen - 11,27; Butan - 0,41; Buten-(1) - 81,78; Buten-(2) - 0,22; Hexen - 5,07; Lösungsmittel - 1,25.

Beispiel 24

Die Herstellung von Buten-(1) erfolgt ähnlicherweise wie in Beispiel 1, man führt jedoch in die aus dem Reaktor abzuleitende Reaktionsmasse Isoamylalkohol ein. Die Zusammensetzung des Destillats nach der Destillation ist wie Folgt, %: Äthylen - 9,94; Butan - 0,40; Buten-(1) - 81,58; Buten-(2) - 0,30; Hexene - 5,77; Lösungsmittel - 2,01.

Beispiel 25

Die Herstellung von Buten-(1) erfolgt ähnlicherweise wie in Beispiel 15, man führt jedoch in die aus dem Reaktor abzuleitende Reaktionsmasse Diäthyläther ein. Die Zusammensetzung des Destillats nach der Destillation ist wie folgt, %: Äthylen - 10,02; Butan - 0,43; Buten--(1) - 81,30; Buten-(2) - 0,45; Hexene - 5,62; Lösungsmittel - 2,00.

Beispiel 26

Die Herstellung von Buten (1) erfolgt unter den dem Beispiel 15 ähnlichen Bedingungen mit Ausnahme dessen, daß man in die Reaktionsmasse Tetrahydrofuran einführt. Die Zusammensetzung des Destillats nach der Destillation ist wie folgt, %: Äthylen - 10,0; Butan - 0,40; Buten-(1) - 81,71; Buten-(2) - 0,29; Hexene - 5,58; Lösungsmittel - 2,02.

Tabelle 1

| Beispiel | Bezeichnung der Komponenten, Masse% | | | | | | Modifizie- rungsmit- tel | Molver- hältnis des Modifi- zie- rungsmit- tels:AlR$_3$ |
|---|---|---|---|---|---|---|---|---|
| | Äthy- len | Bu- tan | Bu- ten- (1) | Bu- ten- (2) | He- xene | Lö- sungs- mittel | | |
| 2 | 10,3 | 0,40 | 80,7 | 0,67 | 5,7 | 2,33 | Methyläthylketon | 0,5:1 |
| 3 | 10,7 | 0,44 | 31,2 | 0,20 | 5,4 | 1,80 | - " - | 5:1 |
| 4 | 11,0 | 0,46 | 81,9 | 0,28 | 5,7 | 0,66 | - " - | 7:1 |
| 5 | 10,5 | 0,41 | 81,6 | 0,75 | 5,6 | 1,09 | Isoamylalkohol | 0,4:1 |
| 6 | 10,4 | 0,43 | 31,8 | 0,25 | 5,4 | 1,72 | - " - | 6:1 |
| 7 | 10,3 | 0,42 | 81,6 | 0,70 | 5,5 | 1,48 | Diäthylenglykol | 0,7:1 |
| 8 | 12,0 | 0,35 | 81,1 | 0,55 | 4,9 | 1,10 | Äthylenglykol | 4:1 |
| 9 | 11,1 | 0,30 | 80,5 | 0,40 | 4,9 | 2,75 | Azetamid | 2:1 |
| 10 | 11,4 | 0,35 | 80,9 | 0,70 | 5,1 | 1,55 | Benzamid | 0,3:1 |

## Industrielle Anwendbarkeit

Die angemeldete Erfindung wird bei der Herstellung von Polybuten, Äthylen-Kopolymer und Buten-(1), Methyl-äthylketon, Essigsäure, Maleinsäureanhydrid, Äthylen-oxid Anwendung finden, wobei als Ausgangsrohstoff Buten--(1) verwendet wird.

PATENTANSPRÜCHE

1. Verfahren zur Herstellung von Buten-(1), das die Dimerisation von Äthylen, die in Gegenwart eines katalytischen Systems Titantetraalkoxid-Trialkylaluminium in einem Kohlenwasserstoff-Lösungsmittel erfolgt, und die anschließende Rektifikation der Reaktionsmasse in Gegenwart eines Modifizierungsmittels vorsieht, d a d u r c h g e k e n n z e i c h n e t, daß man als Modifizierungsmittel eine Verbindung verwendet, die aus der Gruppe der aliphatischen ein- oder zweiwertigen Alkohole, aliphatischer Äther oder zyklischer Äther, aliphatischer Ketone und Karbonsäureamide gewählt wird.

2. Verfahren nach Anspruch 1, d a d u r c h  g e - k e n n z e i c h n e t, daß man bei der Verwendung aliphatischer ein- oder zweiwertiger Alkohole, aliphatischer Äther oder zyklischer Äther, aliphatischer Ketone als Modifizierungsmittel die Rektifikation bei einem Molverhältnis des Modifizierungsmittels zum Trialkylaluminium des katalytischen Systems gleich 1 bis 3 durchführt.

3. Verfahren nach Anspruch 1, d a d u r c h  g e - k e n n z e i c h n e t, daß man bei Verwendung der Karbonsäureamide als Modifizierungsmittel die Rektifikation bei einem Molverhältnis des Modifizierungsmittels zum Trialkylaluminium des katalytischen Systems von 0,25 bis 3,00 durchführt.

# INTERNATIONAL SEARCH REPORT

International Application No   PCT/SU 88/00129

## I. CLASSIFICATION OF SUBJECT MATTER (if several classification symbols apply, indicate all)

According to International Patent Classification (IPC) or to both National Classification and IPC

IPC$^4$: C07C II/08, 2/30

## II. FIELDS SEARCHED

### Minimum Documentation Searched

| Classification System | Classification Symbols |
|---|---|
| IPC$^4$: | C07C II/02 - II/08, 2/26 -2/32 |

### Documentation Searched other than Minimum Documentation to the Extent that such Documents are Included in the Fields Searched

## III. DOCUMENTS CONSIDERED TO BE RELEVANT

| Category | Citation of Document, with indication, where appropriate, of the relevant passages | Relevant to Claim No. |
|---|---|---|
| A | US, A, 3879485, (Gennady Petrovich Belov et al.), 22 April 1975 (22.04.75), see the abstract | I |
| A | FR, AI, 2552079, (INSTITUT FRANCAIS DU PETROLE), 22 March 1985, (22.03.85), see the claims | I |
| A | FR, AI, 2581381, (INSTITUT FRANCAIS DU PETROLE, 7 November 1986 (07.11.86), see the claims | I |
| A | SU, AI, 459451, (V.I.Zhukov et al.) 21 April 1975 (21.04.75), see the claims | I |

* Special categories of cited documents:

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 23 September 1988 (23.09.88) | 5 October 1988 (05.10.88) |

| International Searching Authority | Signature of Authorized Officer |
|---|---|
| ISA/SU | |

Form PCT/ISA/210 (second sheet) (January 1985)